# EUROPEAN PATENT SPECIFICATION

(11) **EP 0 474 754 B1**
(45) Date of publication and mention of the grant of the patent: **13.10.1993**
(21) Application number: 90909199.3
(22) Date of filing: 30.05.1990
(51) Int. Cl.: C02F 11/04, B09B 1/00, C05F 9/00

(54) **A METHOD FOR THE FORCED ANAEROBIC DECOMPOSITION OF WASTE MATERIAL**
VERFAHREN ZUR GESTEUERTEN ANAEROBEN ZERSETZUNG VON ABFALL
PROCEDE DE DECOMPOSITION ANAEROBIE FORCEE DE MATIERE RESIDUELLE

(30) Priority: 30.05.1989 SE 8901940
(43) Date of publication of application: 18.03.1992
(73) Proprietor: VBB VIAK AB, S-102 41 Stockholm (SE)
(72) Inventor: MARELIUS, Kenneth, S-181 48 Lidingö (SE); GRÖNQUIST, Sune, S-114 25 Stockholm (SE)
(74) Representative: Ekelöf, Carl Herman
(86) International application number: SE9000369
(87) International publication number: WO9015031

(56) References cited:
- EP-A- 0 173 667
- EP-A- 0 182 143
- DE-A- 3 109 120
- DE-A- 3 425 788
- US-A- 4 396 402

## Description

The present invention relates to a method for recovering combustible gas, soil substance and fuel fraction from waste that contains organic carbon, by forced anaerobic decomposition of shredded organic waste.

### Background art

It is known that natural anaerobic decomposition of waste and like material containing organic carbon can be achieved. The decomposition process is normally of long duration, due to the fact that the process variables are far from being optimal.

However, many attempts have been made to optimize the anaerobic decomposition of organic material. For instance, Lantmannen no 14 (1979) pages 17-18, describes the anaerobic decomposition of fertilizers with the intention of obtaining methane gas. In this case, the fertilizer is pumped into a container and flows through the container while decomposing and producing methane gas, which is collected in a plastic-fabric container.

US-A-3,586,624 teaches a method in which waste is stacked on a liquid-impermeable support and water is allowed to flow continuously through the waste, with the intention of acceleration decomposition and reducing the risk of fire. It is also possible to leach-out compounds and separate these compounds from the drainage liquid.

US-A-4,323,367 teaches a method in which a container is filled with waste and the gas generated recovered and liquid is recycled.

US-A-4,396,402 teaches a variant of the method just mentioned.

DE-A-27 08 313 teaches a method in which waste is ground to an appropriate size prior to being fed into a digestion vessel. WO-A 84/03694 teaches apparatus for the anaerobic decomposition of waste. This apparatus includes a fermentation container and a conduit system for removing gas from a plastic-sheet covering.

EPA-0 182 143 relates a dump or terminal depository placed on a plastic sheet on the ground and provided with an oxygen sensor which functions to control the gas outtake through valves provided in suction pipes. This publication makes no mention of leaching-water circulation or heating.

DE-C-34 25 785 also teaches a static dump. No mention is made of water-recycling or heating. Gas is removed from a "gas tank" constructed from sheet material. The structural members of the system cannot be reused.

EP-A-0 173 667 teaches a system for withdrawing gas by suction from a static dump, in which the fuel calorific value of the gas removed is increased by washing carbon dioxide from the gas and using the wash water to supply dissolved carbon dioxide in the wash water. The leaching water is not recycled. The gas generation period is said to be about 30 years.

DE-A-34 25 788 teaches a method of constructing a waste dump provided with a passageway system in which three separate pipe systems are used to introduce water, extract gas and remove water and intermediate layers with gas-impervious sheets. The system comprises a fixed or permanent pipe system which results in a higher consumption of pipe material. Gas can be stored in deep chambers protected by the plastic sheets in intermediate layers. No pH-adjustments are made. The publication makes no mention of heat insulation. The gas-extraction pipes and water pipes cannot be reused.

DE-A-31 09 120 relates to a terminal storage arrangement in which carbon dioxide is washed with water from the gas extracted from the deposited material and returned to the arrangement, thereby reducing the nitrogen content of the gas generated, by biological consumption of nitrogen.

Finally, reference is also made to FR-A-1 017 119, which is directed to an arrangement incorporating a removable dome and teaches a process which is self-heating.

All of the aforesaid methods require the provision of permanently mounted apparatus and incur relatively large investments. It is obvious that investment costs are high, due to the relatively long decomposition times involved. Consequently, there is a need for an inexpensive and non-complicated arrangement or system which can be controlled to produce a reproducible result.

Accordingly, the present invention relates to a method for recovering combustible gas from waste material that contains organic carbon, by forced anaerobic decomposition of shredded organic waste, such as domestic waste, food waste and sludge from purification systems. The present invention therefore provides a method for recovering combustible gas, soil and a fuel fraction from waste that contains organic carbon, by forced anaerobic decomposition and classification (sorting), which method is characterized by the steps of shredding the waste to a particle size at which said shredded waste can pass through a sieve having a sieve opening of 20-100 mm; depositing the shredded waste on a liquid-impervious support structure provided with drainage channels such as to form a waste-material bed or heap; covering the bed of shredded waste with a heat-insulating and sealing layer; inserting gas-collecting nozzles through the covering layer, said nozzles being connected through flexible hoses on the upper surface of the bed to a gas-collecting pipe which is located on the upper surface of said bed and through which the gas generated by forced decomposition is extracted by suction; inserting watering nozzles through the covering layer, said nozzles being connected, through flexible hoses located on the upper surface of the bed, to a central watering pipe located on the upper surface of saidbed; forcibly decomposing the waste and, during the decomposition period, controlling the temperature, moisture content, bacteria content and pH of said bed by heating and conditioning the bed of waste.material with leaching water which exits through the drainage channels provided in the support structure and which is heated to a temperature suitable for decomposition and the pH of which is adjusted to a value suitable for decomposition and returned to the bed through the watering nozzles disposed therein; continuing the decomposition process until the generation of gas has terminated; removing the gas-collecting nozzles, gas-collecting pipe, watering nozzles, watering pipe and connecting hoses from the bed of decomposed material, optionally for reuse with another bed of shredded material; and thereafter transporting the bed material to a classifier in which the waste residues are divided into a soil fraction and a fuel fraction.

The heat-insulating layer is preferably earth, peat or some other material which can be mixed to no disadvantage with the waste in the final-use stage or dumping stage. The thickness of the insulating layer is contingent on the insulating properties of the material concerned, the ambient climate and the heat losses permissible through the insulating layer under chosen conditions.

The heat-insulating layer is covered with a moisture-retaining layer, which may either comprise an air-impervious and water-impervious elastic sheet material, such as a plastic material, or a rubber sheet optionally reinforced with fibres. Alternatively, to provide greater mechanical strength, the heat-insulating sheet or layer may consist of a fabric material or natural soil material having a higher proportion of fine particles, or a coarser soil material admixed with bentonite, wood ash or the like. The artificial heat-insulating covering material can be reused upon completion of the decomposition process, whereas a covering layer of natural material can be admixed with the soil derived from the fully-decomposed waste. When the heat-insulating covering comprises a natural material, the thickness of the covering is determined by the nature of the material, atmospheric precipitation, and on the imperviousness of the material to water and air. In order to adjust the air-imperviousness of the material, the surface of said material can be watered at suitable intervals, the amount of water delivered to said material being determined on the basis of the oxygen-content of the gas removed from the decomposing material. The moisture-retaining covering prevents combustible gas from leaking freely to the surroundings, even though it is not gas-impermeable in the true meaning of the expression.

The liquid-impermeable support structure will preferably be almost flat, so that the shredded waste material can be disposed on said support structure and the decomposed material removed therefrom with the aid of simple bucket loaders and like machines. However, the support structure must be configured so that the leaching water generated will flow to a leaching-water channel, from which the leaching water can be carried to a leaching-water reservoir or well. Leaching water can then be taken from the reservoir, reconditioned with respect to temperature, bacteria-content and pH-value, and returned to the decomposing waste material through a watering pipe located centrally on the waste-material pile and through hoses provided with watering nozzles extending from said central pipe. The leaching water is reconditioned by measuring the pH-value of the water and adjusting the water to a pH of about 7. During the initial stage of the decomposition process, the pH of the leaching water will normally lie considerably below 7, due to the release of fatty acids. Consequently, particularly during the initial stage of the decomposition process, a neutralizing base must be supplied through the leaching-water system or by admixing with the waste a neutralizing base, such as calcium hydroxide, calcium carbonate or some other known basic substance which will not impurify the end product with regard to its desired use. An excessively low pH-value will inhibit the generation of methane gas. In the case of anaerobic fermentation processes, decomposition normally takes place at an optimum temperature of about 35-40°C, and consequently the leaching water may be heated prior to being recycled to the waste material bed, at least at low ambient temperatures. The leaching water is preferably heated by heat-exchange with the compressed gas and by combustion of generated combustible gas with the aid of means known to the skilled person, such as a gas-fired boiler or the like. It will be understood, however, that the recycled leaching water can be heated with the aid of other heating sources, such as oil or gas or electrical heating. The leaching water may also be heated through a closed heating loop disposed in the bottom of the bed of waste material, through which heating is effected either with hot water or with the aid of electrical-heating means.

It is also beneficial to ascertain the bacteria content of the waste-material bed, for instance with the aid of known methods, such as by preparing suitable substrate cultivations, dying dried preparations for subsequent microscopic examination, etc.

Combustible gas generated by the decomposition process is removed from the bed by gas-collecting nozzles inserted thereinto, and is withdrawn by suction through a centrally located gas-collecting pipe to the suction side of a gas-pump/compressor/fan or like apparatus. The gas-pump/compressor/fan functions to compress the gas to an overpressure of max 400 kPa and to deliver the pressurized gas to the consumer. The oxygen-content of the withdrawn gas is used as a basis for ascertaining whether or not excessive quantities of air have been drawn in to the bed and therewith having a disturbing effect on the anaerobic decomposition process and that the waste-material bed is nevertheless under subpressure. When the gas withdrawn from the bed contains excessive oxygen, the bed covering is sealed against the ingress of gas, whereas when the oxygen content is excessively low, the amount of gas withdrawn from the waste-material bed is increased, by increasing the pressure in the bed. The extracted and compressed gas is then cooled in a gas cooler and dewatered in a water separator and in a gas drier, the resultant water condensate being removed. Part of the gas recovered is used to reheat the leaching water, the amount of gas required being contingent on ambient temperature, heat losses in the waste-material bed and possible heat losses in watering pipes and nozzles. At low temperatures, it may be necessary to insulate the watering pipes through which reheated leaching water is returned to the bed. The energy-consumption calculated in respect of a waste-material bed for forced anaerobic decomposition of domestic waste in central Sweden is about 10% of the gas-energy taken from the bed.

If desired, the gas can be enriched with regard to desirable components, by absorption of carbon dioxide for instance.

A relatively high moisture content, normally a moisture content of from 70 to 90% by weight, is required to obtain optimum decomposition. The moisture content is controlled by circulation of the leaching water. As a result of using the leaching water to reheat the waste-material bed to an optimum, desired decomposition temperature, the amount of thermal energy supplied through the leaching water will be dependent on the amount of leaching water that must be recycled in order to maintain a satisfactory moisture content of the bed. Thus, in the case of high ambient temperatures, the leaching liquid recycled to the bed need only be heated to a small extent, or need not be heated at all, whereas in the case of low ambient temperatures, it may be necessary to supply more water than is necessary to maintain the moisture content at said desired level, since the recycled water cannot be heated to a temperature at which it would impair the decomposition process when reintroduced into the bed.

Optimum decomposition of the material throughout the whole of the bed also requires a given balance between carbon and nitrogen, estimated at a mole ratio of 60:1 between carbon and nitrogen. When necessary, this balance is controlled by adding carbon and nitrogen to the leaching water in the leaching-water reservoir from which said leaching water is returned to the waste-material bed.

The particle size of the waste is of great importance to the total attack surface of the bacteria on waste constituents, and therewith also on the decomposition rate. The volumetric weight or bulk density of the waste in the established waste-material bed is significant to gas permeability, leaching-water dispersion and heat-transfer in the bed. The waste is processed by shredding the waste to particle sizes of less than 100 mm, preferably from 20 to 50 mm, depending on the type of waste concerned. The volumetric weight or bulk density of the waste in an established waste-material bed is determined with respect to the particle size of the waste and the type of the waste concerned, and may be between 0.4 and 0.7 tonne/m³, which is achieved by compacting the waste or placing the waste in pallets of appropriate height.

An important advantage is afforded by the possibility of using leaching water that is taken from a waste-material bed in which the decomposition process is relatively well established for the purpose of initiating the decomposition process of a newly-established waste-material bed. Consequently, it is obvious that in order to obtain optimum processing of the waste, a number of beds should be established in one and the same location. This will enable a central leaching-water reconditioning plant to be erected for a large number of waste-material beds. Thus, in a waste treatment station fresh beds can be constantly constructed and demolished for reuse or final-dumping of the spent waste subsequent to completion of the decomposition process, which is estimated to take from one to two years. The fuel fraction is separated from the decomposed residues, by rough-sorting of the residues in a helical sieve or the like. When the waste residues are to be used as a soil improving agent, a soil fraction is extracted from said residues with the aid of a vibratory sieve or the like, so as to separate crushed glass, pieces of metal, etc., from the soil extracted.

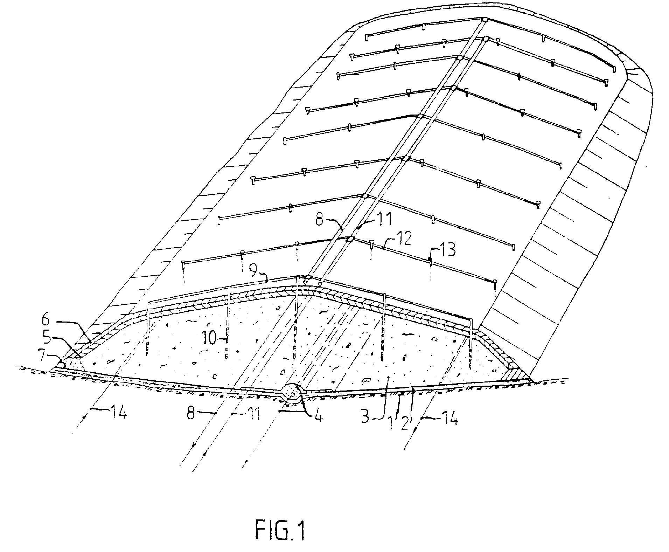

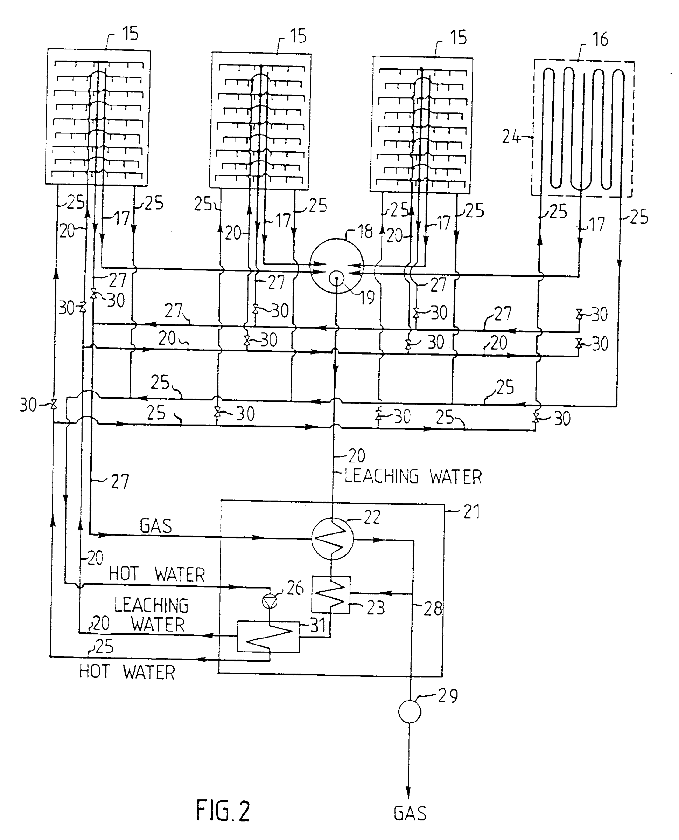

The invention will now be described in more detail with reference to the accompanying drawings, in which Figure 1 is a perspective, sectional view of a waste-material bed or heap; and Figure 2 illustrates schematically a waste treatment station which incorporates arrangements according to the invention. The waste-material bed illustrated in Figure 1 is constructed on smoothed, natural ground which slopes towards one or more central drainage channels 4. The ground surface 1 has been compacted and is covered with a bottom structure made of an essentially water-impervious material 2, which can consist of compressed soil with a sheet of plastic placed on the upper side thereof, asphalt, concrete, rubber or plastic fabric or the like. The bottom structure slopes gently towards the drainage channel or channels. Shredded and compacted waste material, such as domestic waste, garden waste, forest waste, agricultural waste, biomass, organic industrial and commercial waste and like waste material containing organic, decomposible carbon is disposed on the bottom structure 2 such as to form a waste-material bed. As before mentioned, the waste material will preferably have a particle size of between 20 and 100 mm, measured as the quantity of material capable of passing through a screen having a mesh width of 20-100 mm. Subsequent to compaction, the waste material will preferably have a bulk density of 0.4-0.7 tonne/m³. The waste-material bed illustrated in Figure 1 has an elongated configuration, although it will be understood that the geometry of the bed will depend on the conditions prevailing at the site on which the bed is constructed. When the waste treatment station includes a plurality of waste-material beds, all beds will preferably have mutually similar size and shape. The leaching-water drainage channel or channels 4 may consist of one or more perforated drainage pipes made of plastic, rubber or like material, disposed in a drainage ditch which is filled with filtering sand, gravel or like filtering material. The leaching-water drainage system will preferably slope uniformly towards the lower end of the waste-material bed, where the drainage ditch is covered and merges with an imperforate conduit, which in turn leads to a leaching water reservoir or well. The waste is covered with a heat-insulating covering 5 comprising organic material of low thermal conductivity index, such as dry humus, powdered peat or the like. The covering material shall be of a nature which will enable said material to be admixed with the soil derived from the decomposed waste, without having a disturbing effect thereon. The thickness of the heat-insulating covering is determined by the insulating properties of the material concerned, the ambient climate and the permissible heat losses through the insulating covering under optimum climatic conditions. The heat-insulating covering covered with an essentially air-and-water impervious layer consisting of artificial, elastic material such as plastic or rubber-fabric or like material, or by natural material, such as soil which contains a high proportion of fine particles, or a coarser soil admixed with bentonite, wood ash or the like. A perimeter wall 7 of compressed soil is constructed around the waste-material bed as a support for said bed and as a means for preventing water from escaping through the bed perimeter. A gas-collecting pipe 8 and a watering pipe 11 are placed, preferably centrally, on the upper surface of the bed. The pipes 8 and 11 are preferably made of an elastic material, such as polyethylene, glass-fibre reinforced plastic or the like. The pipes, or conduits, are drawn from a gas-pumping station, illustrated in Figure 2. Branching from the main gas conduit are flexible hoses 9, each of which is provided with gas-collecting nozzles 10. The hoses are preferably placed on top of the covering layer and are heat-insulated, as is also the central pipe.

The gas-suction nozzles 10 are made of steel or plastic material and are perforated along the bottom 2/3rds of their lengths. The gas nozzles are inserted through the covering and insulating layers, so that the perforated parts of said nozzles are located in the bed of waste material, and are connected to respective gas-collecting hoses. The number of gas nozzles used and the lengths of respective nozzles is determined by the surface area of the waste-material bed and the suction effect desired. The length dimension of respective gas-suction nozzles is dependent on the position of the nozzle in the bed, and it is ensured in this respect that the suction holes of the nozzle are well distributed in the quantity of waste-material in the bed. The bed is also provided with a centrally positioned watering pipe 11, made of polyethylene, glass-fibre reinforced plastic material or the like, which is dimensioned with respect to the quantity of leaching water to be recycled. Branching from the central watering pipe are hoses 12 provided with watering nozzles 13. Similar to the gas-collecting nozzles, the watering nozzles are made of an appropriate corrosion-resistant metal or plastic material and are perforated at their lower ends and provided with control valves at their upper ends. As before-mentioned, the nozzles are inserted into the upper part of the waste-material bed and the number of nozzles used is determined with regard to the amount of leaching water that must be supplied to the bed and with regard to the need of maintaining a desired moisture content and temperature of the bed.

The illustrated waste-material bed is also provided with a heating loop 14 which functions to circulate heated water and to give-off heat to the bottom layers of the bed. The loop is made of an elastic material, such as polyethylene, glass-fibre reinforced plastic material or the like, and is placed directly on the bottom 2 of the bed. The loop is connected to a circulation pump and to a heat exchanger, which functions to heat the water by heat-exchange with a heating boiler, said boiler being fired with oil or gas, or is electrically heated. The length of the heating loop 14 in the bed and the dimensions of said loop are determined with regard to the heat requirement in the bottom of the bed and by the specific capacity of the loop to transfer thermal energy to the bed. The loop may also comprise an insulated heating cable for electrical heating.

Figure 2 illustrates schematically a plurality of waste-material beds 15 and the site of a future bed 16. Conduits for draining leaching water 17 extend from respective beds to a leaching water reservoir or well 18, where the leaching water is reconditioned. The leaching water is pressed from the reservoir by means of a pump 19, and is carried through a conduit 20 to a gas-pumping station 21, where the leaching water is heated, first by cooling in a compressor with gas coolers 22 and then further in a gas or oil boiler 23. The leaching water is thereafter pressed through a heat exchanger 31, in which part of the thermal energy is transferred to a heating coil and out to the respective waste-material beds, where the water is injected into the waste material through said leaching-water nozzles. For the purpose of further distributing thermal energy within the beds, heating loops 24 are placed in the bottoms of respective beds and in the illustrated future bed 16. The warm water carried by the heating loops is circulated in closed, insulated conduits 25 by means of a circulation pump 26 and obtains thermal energy from the heat exchanger 31. Gas is sucked from the beds through gas nozzles and gas hoses and gas conduits 27 to the compressor (or gas pump or fan) 22 and is pressed therefrom to the consumer, via a pressure conduit 28 and a gas drier 29. Part of the gas is used in the gas boiler to heat the leaching water, prior to returning said water to the waste-material bed. Gas extraction and leaching-water recycling is controlled bedwise with the aid of valves 30 incorporated in control reservoirs or wells.

## Claims

1. A method for recovering combustible gas, soil and a fuel fraction from waste material that contains organic carbon by forced anaerobic decomposition and classification, **characterized** by the steps of shredding the waste to a particle size in which the shredded waste can pass through a screen having a screen opening of 20-100 mm; placing the shredded waste on a liquid-impervious support provided with drainage channels, such as to form a bed on said support; covering the bed of shredded waste-material with a heat-insulating and sealing layer; inserting gas-collecting nozzles through the covering layer, said nozzles being connected, via flexible hoses on the upper surface of the bed, with a gas-collecting pipe arranged on the upper surface of said bed and through which gas-collecting pipe the gas generated by said forced decomposition process is extracted by suction; inserting watering nozzles through the covering layer, said watering nozzles being connected, via flexible hoses on the upper surface of the bed, to a central watering pipe arranged on the upper surface of said bed: subjecting the waste-material in said bed to forced decomposition; controlling such parameters as temperature, moisture content, bacteria content and pH-value during the decomposition period by heating and conditioning leaching water which flows-out through the drainage channels provided in the support structure and which is heated to temperatures of about 35-40 °C appropriate for said decomposition process and the pH of which leaching water is adjusted to pH-values of about 7 appropriate for said decomposition process; recycling the leaching water to the bed through the watering nozzles arranged therein; continuing the decomposition process until gas generation ceases; removing the gas-collecting nozzles, gas-collecting pipes, watering nozzles, watering pipes and connecting hoses from the bed for optional reuse on a fresh waste-material bed, and conveying the bed to a classifying apparatus in which the waste-material residues are divided into a soil fraction and a fuel fraction.

2. A method according to Claim 1, **characterized** in that the heat-insulating covering layer is soil, peat or some other material which can be admixed with the soil fraction of the decomposed waste-material for final use.

3. A method according to Claim 1, **characterized** in that the impervious covering is plastic sheeting, optionally reinforced, or comprises an impervious layer of natural soil material having a high proportion of fine particles, or a coarser soil admixed with bentonite, wood ash or the like and which can be mixed with the soil fraction of the decomposed waste-material to no disadvantage to the final use of said soil fraction.

4. A method according to Claim 1, **characterized** in that the liquid-impervious support structure is sufficiently flat to enable the bed of waste-material to be constructed thereon and removed therefrom with the aid of bucket loaders or like machines.

5. A method according to any one of the preceding claims, **characterized** in that the leaching water is passed to a leaching-water reservoir or well, via drainage pipes; and in that the leaching water is pumped back to the waste-material bed.

6. A method according to Claim 5, **characterized** by measuring the pH of the leaching water and adjusting the pH of the leaching water recycled to the waste-material bed to about 7 and, when necessary, adding carbon and nitrogen to said bed.

7. A method according to Claim 6, **characterized** by heating the leaching water recycled to the bed to a temperature which under steady state conditions will provide a bed temperature of 35-40°C.

8. A method according to Claim 1, **characterized** by measuring the oxygen content of the gas extracted from the bed, and by adjusting the sub-pressure in the bed on the basis of the resultant oxygen values, in a predetermined manner to ascertain sufficient but not excessive air intrusion to the bed.

9. A method according to Claim 8, **characterized** by compressing, cooling and drying the extracted gas, so as to reduce the moisture content of the gas.

10. A method according to Claim 1, **characterized** by using part of the combustible gas extracted from the bed to heat the leaching water to a desired temperature for recycling to said bed.

11. A method according to any one of Claims 5-10, **characterized** by heat-insulating the watering pipe and hoses connected thereto through which heated leaching water is returned to the bed.

12. A method according to Claim 1, **characterized** by supplying additional heat to the bed, by circulating heated leaching water through a heating loop disposed in the bottom of the bed.

## Patentansprüche

1. Verfahren zur Wiedergewinnung von brennbarem Gas, Erde und einer Brennstoff-Fraktion aus Abfallmaterial, das organischen Kohlenstoff enthält, durch zwangsweise anaerobe Zersetzung und Klassifizierung, gekennzeichnet durch die Schritte: Zerkleinern des Abfalls auf eine Partikelgröße, bei der der zerkleinerte Abfall durch ein Sieb mit einer Sieböffnung von 20-100 mm hindurchgehen kann; Anordnen des zerkleinerten Abfalls auf einem mit Drainagekanälen versehenen flüssigkeitsundurchlässigen Träger, so daß sich auf dem Träger ein Beet bildet; Bedecken des Beetes von zerkleinertem Abfallmaterial mit einer wärmeisolierenden und abdichtenden Schicht; Einführen von Gassammeldüsen durch die Abdeckschicht, wobei die genannten Düsen über flexible Schläuche auf der oberen Fläche des Beetes mit einem auf der oberen Fläche des genannten Beetes angeordneten Gassammelrohr verbunden sind und durch dieses Gassammelrohr das durch den genannten zwangsweisen Zersetzungsprozeß erzeugte Gas durch Absaugen extrahiert wird; Einführen von Bewässerungsdüsen durch die Abdeckschicht, wobei die genannten Bewässerungsdüsen über flexible Schläuche auf der oberen Fläche des Beetes mit einem auf der oberen Fläche des genannten Beetes angeordneten zentralen Bewässerungsrohr verbunden sind; Behandeln des Abfallmaterials in dem genannten Beet durch zwangsweise Zersetzung; Regelung von Parametern wie Temperatur, Feuchtigkeitsgehalt, Bakteriengehalt und pH-Wert während der Zersetzungsperiode durch Erwärmen und Konditionieren von Sickerwasser, das durch die in der Trägerstruktur vorgesehenen Drainagekanäle ausfließt, und das auf für den genannten Zersetzungsprozeß geeignete Temperaturen von ungefähr 35-40°C erwärmt wird und der pH dieses Sickerwassers auf für den genannten Zersetzungsprozeß geeignete pH-Werte von ungefähr 7 eingestellt wird; Rückführung des Sickerwassers in das Beet durch die darin angeordneten Bewässerungsdüsen; Fortsetzen des Zersetzungsprozesses bis die Gaserzeugung aufhört; Entfernen der Gassammeldüsen, Gassammelrohre, Bewässerungsdüsen, Bewässerungsrohre und Verbindungsschläuche aus dem Beet zur möglichen Wiederverwendung auf einem frischen Abfallmaterialbeet und Fördern des Beetes zu einem Klassifiziergerät, in dem die Abfallmaterialrückstände in eine Erdfraktion und eine Brennstoff-Fraktion geteilt werden.

2. Verfahren nach Anspruch 1, dadurch gekennzeichnet, daß die wärmeisolierende Abdeckschicht Erde, Torf oder ein anderes Material ist, das mit der Erdfraktion des zersetzten Abfallmaterials für die endgültige Verwendung vermischt werden kann.

3. Verfahren nach Anspruch 1, dadurch gekennzeichnet, daß die undurchlässige Abdeckung eine, gegebenenfalls verstärkte, Plastikplane ist oder eine undurchlässige Schicht von natürlichem Erdmaterial mit einem hohen Anteil von feinen Partikeln oder eine grobere Erde vermischt mit Bentonit, Holzasche oder dergleichen umfaßt, und die mit der Erdfraktion des zersetzten Abfallmaterials ohne Nachteile für die endgültige Verwendung der genannten Erdfraktion gemischt werden kann.

4. Verfahren nach Anspruch 1, dadurch gekennzeichnet, daß die flüssigkeitsundurchlässige Trägerstruktur ausreichend flach ist, um zu ermöglichen, daß das Beet von Abfallmaterial mit Hilfe von Schaufelladern oder dergleichen Maschinen darauf gebaut und davon entfernt werden kann.

5. Verfahren nach einem der vorhergehenden Ansprüche, dadurch gekennzeichnet, daß das Sickerwasser über Drainagerohre in ein Sickerwasserreservoir oder einen Brunnen geführt wird; und daß das Sickerwasser zum Abfallmaterialbeet zurückgepumpt wird.

6. Verfahren nach Anspruch 5, gekennzeichnet durch Messung des pH des Sickerwassers und Einstellen des pH des zum Abfallmaterialbeet rückgeführten Sickerwassers auf ungefähr 7 und, wenn nötig, Zusatz von Kohlenstoff und Stickstoff in das genannte Beet.

7. Verfahren nach Anspruch 6, gekennzeichnet durch Erwärmen des in das Beet rückgeführten Sickerwassers auf eine Temperatur, die unter stationären Zustandsbedingungen zu einer Beettemperatur von 35-40°C führt.

8. Verfahren nach Anspruch 1, gekennzeichnet durch Messung des Sauerstoffgehaltes des vom Beet extrahierten Gases und durch Einstellen des Unterdruckes im Beet auf Basis der erhaltenen Sauerstoffwerte auf eine bestimmte Weise, um ausreichende, aber nicht überschüssige Luftzufuhr im Beet sicherzustellen.

9. Verfahren nach Anspruch 8, gekennzeichnet durch Komprimieren, Abkühlen und Trocknen des extrahierten Gases, um den Feuchtigkeitsgehalt des Gases zu reduzieren.

10. Verfahren nach Anspruch 1, gekennzeichnet durch Verwendung eines Teils des aus dem Beet extrahierten brennbaren Gases zum Erwärmen des Sickerwassers auf eine gewünschte Temperatur zur Rückführung in des genannte Beet.

11. Verfahren nach einem der Ansprüche 5-10, gekennzeichnet durch Wärmeisolieren des Bewässerungsrohrs und damit verbundenen Schläuche, durch die erwärmtes Sickerwasser zum Beet zurückgeleitet wird.

12. Verfahren nach Anspruch 1, gekennzeichnet durch Zufuhr zusätzlicher Wärme zum Beet durch Zirkulieren erwärmten Sickerwassers durch eine unten im Beet angeordnete Heizungsschleife.

## Revendications

1. Procédé de récupération de gaz inflammable, de sol et d'une fraction de combustible à partir d'une matière résiduelle qui contient du carbone organique, par décomposition anaérobie forcée et classification, caractérisé par les étapes de : broyage du résidu en une grosseur de particule avec laquelle le résidu broyé peut passer à travers un tamis ayant une ouverture de tamis de 20-100 mm; placement du résidu broyé sur un support imperméable aux liquides muni de canaux de drainage, de manière à former un lit sur ledit support; couverture du lit de matière résiduelle avec une couche d'isolation thermique et d'étanchéité; insertion de buses collectrices de gaz à travers la couche de couverture, lesdites buses étant raccordées, par des tuyaux flexibles sur la surface supérieure du lit, à une canalisation collectrice de gaz arrangée sur la surface supérieure dudit lit, le gaz généré par ledit procédé de décomposition forcée étant extrait par aspiration à travers cette canalisation collectrice de gaz; insertion de buses d'arrosage à travers la couche de couverture, lesdites buses d'arrosage étant raccordées, par des tuyaux flexibles sur la surface supérieure du lit, à une canalisation centrale d'arrosage arrangée sur la surface supérieure dudit lit; soumission de la matière résiduelle dans ledit lit à une décomposition forcée; contrôle de paramètres tels que la température, l'humidité, la teneur en bactéries et la valeur du pH durant la période de décomposition, par le chauffage ainsi que par le conditionnement d'eau de lixiviation qui s'écoule à travers les canaux de drainage fournis dans la structure du support et qui est chauffée à des températures d'environ 35-40°C, appropriées pour ledit procédé de décomposition, le pH de cette eau de lixiviation étant ajusté à des valeurs de pH d'environ 7, appropriées pour ledit procédé de décomposition; recyclage de l'eau de lixiviation vers le lit à travers les buses d'arrosage arrangées dans celui-ci; continuation du procédé de décomposition jusqu'à ce que la génération de gaz cesse; enlèvement des buses collectrices de gaz, des canalisations collectrices de gaz, des buses d'arrosage, des canalisations d'arrosage et des tuyaux de raccordement du lit, pour une réutilisation optionnelle sur un lit de matière résiduelle fraîche; et transport du lit vers un appareil de classification dans lequel les produits de la matière résiduelle sont divisés en une fraction de sol et une fraction de combustible.

2. Procédé selon la revendication 1, caractérisé en ce que la couche de couverture d'isolation thermique est du sol, de la tourbe ou une autre matière quelconque qui peut être ajoutée à la fraction de sol de la matière résiduelle décomposée pour l'utilisation finale.

3. Procédé selon la revendication 1, caractérisé en ce que la couverture imperméable est une feuille en matière plastique, optionnellement renforcée, ou comprend une couche imperméable de matière naturelle du sol ayant une proportion élevée de particules fines, ou encore du sol plus grossier ajouté à de la bentonite, des cendres de bois ou autre, qui peuvent être mélangés à la fraction de sol de la matière résiduelle décomposée sans désavantage quant à l'utilisation finale de ladite fraction de sol.

4. Procédé selon la revendication 1, caractérisé en ce que la structure du support imperméable aux liquides est suffisamment plane pour autoriser que le lit de matière résiduelle soit construit sur celui-ci et enlevé de dessus celui-ci à l'aide de rotopelles ou d'autres machines.

5. Procédé selon l'une quelconque des revendications précédentes, caractérisé en ce que l'eau de lixiviation passe dans un réservoir ou un puits d'eau de lixiviation, par des canalisations de drainage; et en ce que l'eau de lixiviation est pompée de nouveau vers le lit de matière résiduelle.

6. Procédé selon la revendication 5, caractérisé par la mesure du pH de l'eau de lixiviation, l'ajustement du pH de l'eau de lixiviation recyclée vers le lit de matière résiduelle à environ 7 et, lorsque nécessaire, l'addition de carbone et d'azote audit lit.

7. Procédé selon la revendication 6, caractérisé par le chauffage de l'eau de lixiviation recyclée vers le lit à une température qui, dans des conditions stables, fournit une température du lit de 35-40°C.

8. Procédé selon la revendication 1, caractérisé par la mesure de la teneur en oxygène du gaz extrait du lit, et par l'ajustement de la sous-pression dans le lit sur la base des valeurs résultantes de l'oxygène, d'une manière prédéterminée, pour assurer une entrée d'air suffisante mais non excessive vers le lit.

9. Procédé selon la revendication 8, caractérisé par la compression, le refroidissement et le séchage du gaz extrait, de manière à réduire l'humidité du gaz.

10. Procédé selon la revendication 1, caractérisé par l'utilisation d'une partie du gaz inflammable extrait du lit pour chauffer l'eau de lixiviation jusqu'à une température désirée pour le recyclage vers ledit lit.

11. Procédé selon l'une quelconque des revendications 5-10, caractérisé par l'isolation thermique de la canalisation d'arrosage et des tuyaux qui y sont raccordés à travers lesquels l'eau de lixiviation chauffée est renvoyée vers le lit.

12. Procédé selon la revendication 1, caractérisé par la fourniture de chaleur additionnelle au lit, par la circulation d'eau de lixiviation chauffée à travers une boucle de chauffage disposée dans le fond du lit.
